# EUROPÄISCHE PATENTSCHRIFT

(11) **EP 2 173 411 B1**
(45) Veröffentlichungstag und Bekanntmachung des Hinweises auf die Patenterteilung: **11.11.2015**
(21) Anmeldenummer: 08785160.6
(22) Anmeldetag: 28.07.2008
(51) Int. Cl.: A61M 5/158, A61M 5/32, A61M 39/02

(54) **VORRICHTUNG ZUM APPLIZIEREN EINER KANÜLE**
DEVICE FOR ADMINISTERING A CANNULA
DISPOSITIF DE MISE EN PLACE D'UNE CANULE

(30) Priorität: 09.08.2007 DE 202007011154 U
(43) Veröffentlichungstag der Anmeldung: 14.04.2010
(73) Patentinhaber: Mantsch, Christian, 32427 Minden (DE); Stumpp, Uwe, 78665 Frittlingen (DE)
(72) Erfinder: STUMPP, Uwe, 78665 Frittlingen (DE)
(74) Vertreter: Ter Meer Steinmeister & Partner
(86) Internationale Anmeldenummer: PCT/EP2008/006211
(87) Internationale Veröffentlichungsnummer: WO 2009/018937

(56) Entgegenhaltungen:
- EP-A- 1 749 547
- WO-A-02/41932
- WO-A-2005/079441
- US-A- 5 147 303
- US-A- 5 246 427
- US-B1- 6 238 375
- US-B1- 6 969 372

## Beschreibung

Die Erfindung betrifft eine Vorrichtung zum Applizieren einer Kanüle.

Insbesondere betrifft die Erfindung eine Vorrichtung zum perkutanen Einführen einer Kanüle in einen implantierten Port eines Portkathetersystems. Ein solcher Port weist typischerweise ein Verschlußelement beispielsweise aus Silikon auf, welches mit der Kanüle durchstochen wird. Das Portkathetersystem kann beispielsweise ein venöser Katheter für die Chemotherapie sein.

US 6,969,372 D1 beschreibt eine Vorrichtung zum automatischen Zurückziehen einer Nadel in ein Gehäuse. Ein Deckel des Gehäuses ist an einem hinteren Ende schwenkbar am Gehäuse befestigt und am vorderen Ende mit einem Vorsprung an einer Einkerbung der Innenseite des Gehäuses verrastet. Durch Einwärtsdrücken eines Drückers wird die Verriegelung gelöst, und der Deckel wird durch eine auf der Nadel geführte Sprungfeder aufgeklappt. Dabei wird die am vorderen Ende des Deckels abwärts gerichtete Nadel in das Gehäuse eingezogen und seitlich verschwenkt, wobei eine Spitze der Nadel in einer Ecke des Gehäuses verborgen wird.

US 6,238,375 B1 beschreibt ein mehrteiliges Gehäuse für eine Nadel für einen Venenzugangsport. Eine Feder spannt einen oberen Gehäuseteil gegenüber einem unteren Gehäuseteil vor, und eine Feder spannt eine Verschlusskappe in eine geschlossene Position über der unterseitigen Öffnung des unteren Gehäuseteils vor. Die Federn üben jeweils nur minimale Vorspannkräfte aus. Zur Anwendung wird eine Nadel an dem Gehäuse angebracht, die Verschlussklappe wird geöffnet, und das Gehäuse wird zusammengeschoben, um die Nadel in den Venenzugangsport einführen zu können. Die Nadel wird dann durch die Verschlusskräfte eines Septums des Ports gehalten. Nach dem Herausziehen der Nadel verschließt die Klappe dann automatisch die untere Öffnung des Gehäuses.

WO 2005/079441 A2 beschreibt eine Vorrichtung zum Verabreichen eines Impfstoffes. Zum Zurückziehen der Injektionsnadel wird ein seitlich angeordneter Knopf einwärts gedrückt, und eine Feder fährt einen Nadelschlitten in eine obere Position, wobei die Nadel in das Gehäuse eingezogen wird. Die Vorrichtung umfasst eine Verschlussscheibe, die mittels eines Knaufs mit einem Finger vor die untere Gehäuseöffnung gedreht werden kann, um ein Ausfahren der Nadel zu verhindern.

WO 02/41932 A2 beschreibt eine Vorrichtung zur peripheren Blutgefäßkatheterisierung. Die Vorrichtung umfasst einen federnden Knopf, der drei Positionen einnehmen kann: eine anfängliche Verriegelungsposition, eine Freigabeposition, und eine Abschirmungsposition. Durch Niederdrücken des Knopfes wird die Freigabeposition eingenommen, in der eine Feder eine Nadeleinheit in eine äußerste proximale Position zurückzieht. Nach Loslassen des Knopfes federt ein Riegel in eine Abschirmungsposition, in der er als Schild ein Austreten der distalen Nadelspitze in distale Richtung verhindert.

US 5 147 303 A beschreibt eine Spritze, die innerhalb einer Hülse gegen eine Vorspannung einer Druckfeder mittels einer Verriegelung gehalten wird. Die Verriegelung greift in ein oberes Ende der Druckfeder ein. Am unteren Ende der Hülse ist eine Öffnung für die Nadel, die nach einem Zurückziehen der Nadel in die Hülse durch eine umklappbare Scheibe verschlossen werden kann, welche durch eine Feder vorgespannt ist. Wenn nach dem Benutzen der Spritze die Spritze entsorgt werden soll, wird die Spritze weit genug niedergedrückt, um die Verriegelung außer Eingriff mit der Druckfeder zu bringen, so dass die Druckfeder sich ausdehnt und die Spritze relativ zur Hülse anhebt, um die Nadel in die Hülle zurückzuziehen.

Es ist eine Vorrichtung zum Applizieren einer Kanüle bekannt, bei der nach dem Punktieren des Ports eines Katheters mit der Kanüle, und nachdem beispielsweise ein Medikament durch die Kanüle injiziert wurde, zum Herausziehen der Kanüle die Vorrichtung am Patienten zurückgehalten wird, um die Kanüle in die Vorrichtung zurückzuziehen. Das Zurückziehen der Kanüle in die Vorrichtung erfolgt, um die Spitze der Kanüle zu verbergen und so die Verletzungsgefahr zu verringern. Der dabei auf die Vorrichtung auszuübende Gegendruck kann dazu führen, daß die Spitze der Kanüle vor dem Herausziehen leicht gekrümmt wird, so daß beim Herausziehen das Verschlußelement des Ports durch die verformte Spitze beschädigt wird. Beispielsweise könnten kleine Teile des Verschlußelements herausgeschnitten werden, so daß das Verschlußelement bei wiederholter Punktierung undicht wird.

Aufgabe der Erfindung ist es daher, eine Vorrichtung der eingangs genannten Art zu schaffen, die nach dem Punktieren eines implantierten Ports ein leichtes und möglichst zerstörungsfreies Herausziehen der Kanüle ermöglicht.

Diese Aufgabe wird erfindungsgemäß durch eine Vorrichtung nach Anspruch 1 gelöst.

Vorzugsweise ist das Zugelement in der gebrauchsfertigen Stellung an dem Basiselement angeordnet, insbesondere an diesem gehalten. Vorzugsweise ist das Zugelement an einem Deckel der Vorrichtung angeordnet.

Da erfindungsgemäß die Vorspanneinrichtung über das Zugelement die Kanüle zurückzieht, kann währenddessen das Basiselement in unveränderter Position festgehalten werden, ohne daß ein Gegendruck in Richtung der Einstichstelle ausgeübt werden muß. Dadurch ist sichergestellt, daß die Spitze der Kanüle nicht gekrümmt oder verbogen wird. Somit wird die Kanüle geschont, und es wird eine Beschädigung des Ports vermieden. Außerdem kann die erfindungsgemäße Vorrichtung mit einer Hand bedient werden, da keine zweite Hand zum Erfassen der Kanüle benötigt wird. Dies vereinfacht den Arbeitsablauf erheblich.

Die Vorrichtung wird angewendet, indem in der gebrauchsfertigen Stellung die mit ihrer Spitze über das Basiselement hinausragende Kanüle durch die Haut des Patienten in den darunter implantierten Port eingeführt wird. Das Basiselement der Vorrichtung kommt dabei beispielsweise zur Anlage auf der Haut des Patienten und kann dann zum Abstützen der Vorrichtung auf der Haupt des Patienten dienen. Nachdem beispielsweise über die Kanüle ein Medikament injiziert wurde, wird zum Herausziehen der Kanüle das Basiselement fest gehalten, beispielsweise durch seitliches Erfassen des Basiselements, und es wird die Vorspanneinrichtung gelöst. Dadurch wird automatisch die Spitze der Kanüle in das Basiselement zurückgezogen.

Vorteilhafte Ausgestaltungen der Erfindung ergeben sich aus den Unteransprüchen.

Vorzugsweise ist die Vorrichtung für Einhandbedienung zum Festhalten des Basiselements und Lösen der Vorspanneinrichtung, während das Basiselement auf der Haut eines Patienten anliegt, ausgelegt. Das heißt, die Nadel kann herausgezogen werden, während gleichzeitig das Basiselement festgehalten wird, wofür nur eine Hand benötigt wird. Im Gegensatz dazu ist bei herkömmlichen Vorrichtungen zum Applizieren einer Kanüle stets eine Bedienung mit zwei Händen erforderlich, da mit einer Hand die Kanüle herausgezogen wird, indem beispielsweise ein Griffelement erfaßt und von einem Basiselement in einer Richtung parallel zur Hauptachse der Kanüle weg gezogen wird, während die Vorrichtung mit einer zweiten Hand am Patienten zurückgehalten wird.

Erfindungsgemäß weist die Vorrichtung wenigstens ein Betätigungselement zum Lösen der Vorspanneinrichtung auf, wobei das Betätigungselement durch Bewegen, insbesondere Drücken, des Betätigungselements in eine Richtung quer zur Kanüle betätigt wird. Somit wird zum Lösen der Vorspanneinrichtung kein Druck in Richtung des Ports ausgeübt. Erfindungsgemäß wird insbesondere das Betätigungselement durch Drücken des Betätigungselements in Richtung zur Mittelachse der Kanüle betätigt. Insbesondere handelt es sich bei dem erwähnten Drücken um ein Drücken mit einer Hand, d.h. einem oder mehreren Fingern.

Vorzugsweise ist das Basiselement durch Erfassen an dem Betätigungselement und an einer gegenüberliegenden Seite des Basiselements festhaltbar, insbesondere mit nur einer Hand festhaltbar.

Die Vorspanneinrichtung ist vorzugsweise lösbar durch Drücken des Betätigungselements durch seitliches Erfassen des Basiselements. Vorzugsweise ist das Betätigungselement in eine Richtung quer zur Kanüle drückbar, um die Vorspanneinrichtung zu lösen. Mit anderen Worten ist die Vorspanneinrichtung lösbar, indem durch seitliches Erfassen des Basiselements wenigstens in einem Bereich des Betätigungselements und Druckausübung auf das Betätigungselement das Betätigungselement in eine Richtung quer zur Kanüle gedrückt wird.

Es wird also die Vorrichtung mit in die Haut des Patienten eingeführter Kanüle mit einer Hand seitlich erfaßt, d.h. quer zur Kanüle auf zwei einander gegenüberliegenden Seiten, beispielsweise mit zwei Fingern, etwa Zeigefinger und Daumen, und durch ein Zusammendrücken wird die Vorspanneinrichtung gelöst.

Vorzugsweise ist an dem Betätigungselement ein Riegelelement angeordnet, und an dem Zugelement ist ein Gegenstück zu dem Riegelelement angeordnet, welches Gegenstück in der gebrauchsfertigen Stellung an dem Riegelelement gehalten ist, und das Lösen der Vorspanneinrichtung erfolgt, indem das Riegelelement das Gegenstück freigibt. Beispielsweise wird das an dem Betätigungselement angeordnete Riegelelement durch Drücken und Verschieben des Betätigungselements in eine Richtung zur Mittelachse der Kanüle hin von seinem Gegenstück gelöst. Besonders bevorzugt bildet das Betätigungselement das Riegelelement oder mehrere Riegelelemente für entsprechende Gegenstücke des Zugelements. Indem zum Lösen des Riegelelements von seinem Gegenstück lediglich eine Bewegung quer zur Richtung der Kanüle und somit quer zur Kraftrichtung der Vorspanneinrichtung erfolgt, läßt sich die Vorspanneinrichtung besonders leicht lösen, und es ist hierzu keine Kraftausübung in Richtung des Ports notwendig.

Vorzugsweise ist das Betätigungselement plattenförmig und ist an dem Basiselement gehalten, und in der gebrauchsfertigen Stellung ist das Zugelement an dem Betätigungselement verriegelt, wobei die Verriegelung durch Bewegen des Betätigungselements in einer Richtung quer zur Kanüle lösbar ist. Dadurch ergibt sich ein besonders einfacher und flacher Aufbau der Vorrichtung.

Vorzugsweise ist das Betätigungselement an dem Basiselement in einer Richtung quer zur Kanüle bewegbar, insbesondere verschiebbar, gehalten, und in der gebrauchsfertigen Stellung hintergreift wenigstens ein Riegelelement des Zugelements ein Riegelelement des Betätigungselements in Form eines Bereichs des Betätigungselements, welcher Bereich geformt ist, um durch Bewegen des Betätigungselements in einer Richtung quer zur Kanüle außer Eingriff mit dem Riegelelement des Zugelements gebracht zu werden. Indem so der Bereich des Betätigungselements das Riegelelement des Zugelements freigibt, ist die Vorspanneinrichtung lösbar.

Vorzugsweise befindet sich ein jeweiliger, in der gebrauchsfertigen Stellung von einem jeweiligen Riegelelement des Zugelements hintergriffener Bereich an einem Rand des Betätigungselements, insbesondere an einem seitlichen Rand, welcher Rand quer zu einer Verschiebungsrichtung des Betätigungselements verläuft und/oder an welchem Rand das Betätigungselement an dem Basiselement verschiebbar gehalten ist.

Besonders bevorzugt sind mehrere derartige Riegelelemente des Zugelements in der gebrauchsfertigen Stellung um die Kanüle herum angeordnet, beispielsweise vier Riegelelemente an gegenüberliegenden Rändern eines im wesentlichen rechteckförmigen Betätigungselements. Vorzugsweise ist an dem jeweiligen Bereich eine Aussparung zum Hindurchtreten des jeweiligen Riegelelements, um dasselbe freizugeben, vorgesehen.

Indem zum Lösen der Riegelelemente des Betätigungselements von ihren Gegenstücken des Zugelements lediglich eine Bewegung des Betätigungselements quer zur Richtung der Kanüle und somit quer zur Kraftrichtung der Vorspanneinrichtung benötigt wird, kann die Vorspanneinrichtung besonders leicht gelöst werden. Zudem genügt es, ein einziges verschiebbares Betätigungselement vorzusehen, welches beispielsweise unmittelbar durch Drücken gegen seinen am Umfang der Vorrichtung angeordneten äußeren Rand verschiebbar ist.

In einer weiteren Ausführungsform ist das wenigstens eine Betätigungselement an einem Bügel angeordnet, der in der Nähe eines äußeren Randes des Basiselements verläuft und an seinen Enden mit dem Basiselement verbunden ist. Insbesondere erstreckt der Bügel sich halbringförmig um das Basiselement herum. Dadurch läßt sich der Bügel mit dem Betätigungselement besonders gut erfassen, und der Bügel sorgt außerdem durch seine beidseitige Verbindung mit dem Basiselement für eine gewisse Führung des Betätigungselements.

Optional sind zwei Betätigungselemente zum Lösen der Vorspanneinrichtung einander gegenüberliegend an dem Basiselement angeordnet. Dadurch sind die Betätigungselemente besonders gut zum Festhalten des Basiselements mit einer Hand geeignet, und beim gleichzeitigen Zusammendrücken der beiden Betätigungselemente wird ein seitliches Verschieben der Kanüle oder eine Ausübung einer Querkraft auf die Kanüle wirksam verhindert. Die beiden Bügel der Befestigungselemente bilden beispielsweise einen das Basiselement umfassenden Ring.

Erfindungsgemäß weist die Vorrichtung wenigstens ein Verschlußelement zum Verbergen der Spitze der Kanüle auf, wenn diese in das Basiselement zurückgezogen wurde. Optional kann das Verschlußelement durch Federkraft in eine die Spitze verbergende Stellung bewegbar sein. Beispielsweise kann das Verschlußelement an einem Federelement angeordnet sein und in der Gebrauchsfertigen Stellung vorgespannt sein und durch Betätigen des Betätigungselementes auslösbar sein, um eine die in das Basiselement zurückgezogene Spitze verbergende Stellung einzunehmen. Beispielsweise kann das Verschlußelement in der gebrauchsfertigen Stellung seitlich gegen die Kanüle vorgespannt sein. Wenn die Spitze der Kanüle an dem Verschlußelement vorbei in das Basiselement zurückgezogen wird, wird so automatisch das Verschlußelement ausgelöst. Das Verschlußelement kann beispielsweise ein plattenförmiges Element sein, welches einstückig mit einer seitlich abstehenden Blattfeder ausgebildet ist, wobei durch Lösen der Vorspannung das plattenförmige Element seitlich und quer zur Kanüle bewegbar ist.

Besonders bevorzugt bildet das Betätigungselement das Verschlußelement. Vorzugsweise weist das Betätigungselement eine Öffnung für die Kanüle in der gebrauchsfertigen Stellung auf. Indem das Betätigungselement unmittelbar das Verschlußelement bildet, ergibt sich ein besonders einfacher und sicher zu betätigender Aufbau.

Das Verschlußelement ist vorzugsweise in der Nähe einer Unterseite des Basiselements angeordnet, also in der Nähe der zur Spitze der Kanüle gerichteten Seite. Dies ist die der Haut des Patienten zugewandte Seite. Beispielsweise weist die Vorrichtung zwei Verschlußelemente auf gegenüberliegenden Seiten auf, die in eine Position versetzbar sind, in der ihre vorderen Enden sich vor der Spitze befinden. Durch das wenigstens eine Verschlußelement werden Verletzungen an der Spitze der Kanüle nach dem Gebrauch der Vorrichtung ausgeschlossen. Vorzugsweise ist das Verschlußelement verschiebbar an dem Basiselement gehalten.

Erfindungsgemäß ist das Verschlußelement derart an dem Basiselement gehalten, daß durch Drücken des Betätigungselements in der Richtung quer zur Kanüle das Verschlußelement vor die in das Basiselement zurückgezogene Spitze der Kanüle bewegbar ist. Dies erfolgt beispielsweise, indem das Betätigungselement einen Stößel aufweist oder mit einem Stößel verbunden ist oder ihn steuert, welcher Stößel das Verschlußelement verschiebt. Beispielsweise kann der Stößel an dem oben erwähnten Riegelelement angeordnet oder mit diesem verbunden sein. Dadurch kann mit einer einzigen Bewegung des Betätigungselements die Vorspanneinrichtung gelöst und, nach dem sofort erfolgenden Zurückziehen der Kanüle, das Verschlußelement vor die Spitze bewegt werden.

Besonders bevorzugt bildet wiederum das Betätigungselement das Verschlußelement.

Vorzugsweise weist die Vorrichtung eine Verriegelungseinrichtung zum Verriegeln des Verschlußelements in der die Spitze verbergenden Stellung auf. Die Verriegelungseinrichtung besteht vorzugsweise aus Rastelementen, die an dem Betätigungselement und an dem Basiselement vorgesehen sind. Beispielsweise kann das verschiebbar an dem Basiselement gehaltene Betätigungselement quer zur Verschiebungsrichtung vorspringende Rastnasen aufweisen, die in der die Spitze verbergenden Stellung in Rastkerben des Basiselements eingreifen. Weiter kann die Verriegelungseinrichtung beispielsweise aus einem mit dem Betätigungselement verbundenen oder an diesem angeordneten zweiten Riegelelement und einer diesem zugeordneten Aufnahme am Basiselement bestehen. Das Riegelelement kann beispielsweise an dem erwähnten Stößel angeordnet sein. Durch die Verriegelungseinrichtung ist sichergestellt, daß die Spitze der Kanüle nicht wieder freigelegt wird.

Durch die Verriegelung mittels Rastelementen kann das Verschlußelement nicht wieder zurückgeschoben werden.

Vorzugsweise ist das Betätigungselement plattenförmig und ist verschiebbar an dem Basiselement gehalten, bildet das oben genannte Verschlußelement, und steht in der gebrauchsfertigen Stellung in Eingriff mit den oben genannten Riegelelementen des Zugelements, wobei die Vorspanneinrichtung durch Verschieben des Betätigungselements lösbar ist und durch weiteres Verschieben des Betätigungselements ein Verschlußbereich des Betätigungselements vor die in das Basiselement zurückgezogene Spitze der Kanüle bewegbar ist. Indem durch Drücken auf das Betätigungselement zunächst die Vorspanneinrichtung gelöst wird, so daß die Kanüle zurückgezogen wird, und dann die Spitze der Kanüle verborgen wird und gegebenenfalls eine Verriegelung des Betätigungselements in der die Spitze verbergenden Stellung erfolgt, wird durch eine einzige geradlinige Bewegung des Betätigungselements sowohl die Vorspanneinrichtung gelöst als auch die dadurch zurückgezogene Kanüle gesichert. Es ergibt sich somit ein besonders funktionssicherer Aufbau der Vorrichtung und eine sehr sichere Handhabung.

Optional weist die Vorrichtung wenigstens eine Zunge auf, die quer zur Kanüle in das Basiselement hineinragt und durch Betätigen des Betätigungselements vorschiebbar ist, wobei die Zunge das Riegelelement aufweist, einen Stößel zum Bewegen des Verschlußelements vor die in das Basiselement zurückgezogene Spitze der Kanüle bildet und einen Bestandteil der Verriegelungseinrichtung zum Verriegeln des Verschlußelements aufweist, mit welchem Bestandteil die Zunge an einer zugeordneten Aufnahme am Basiselement verrastbar ist. Vorzugsweise ist die Zunge mit dem Betätigungselement verbunden oder ist ein Teil desselben. Die Verriegelungseinrichtung besteht somit aus ihrem Bestandteil, welcher einen Teil der Zunge bildet, und der zugeordneten Aufnahme am Basiselement. Vorzugsweise ragt die Zunge in Richtung auf die Mittelachse der Kanüle in das Basiselement hinein. Indem durch Drücken auf das Betätigungselement die Zunge in diese Richtung bewegt wird, wird durch eine einzige, geradlinige Bewegung zuerst die Vorspanneinrichtung gelöst, so daß die Kanüle zurückgezogen wird, und dann das Verschlußelement vor die Spitze der Kanüle geschoben, wobei die Zunge am Basiselement verrastet wird. Dadurch kann das Verschlußelement nicht wieder zurückgeschoben werden. Es ergibt sich somit wiederum eine sehr einfache Handhabung der Vorrichtung. Dieser Aufbau der Vorrichtung ist ebenfalls besonders funktionssicher.

Es erfolgt wiederum durch Drücken auf das Betätigungselement durch eine einzige, geradlinige Bewegung das Lösen der Vorspanneinrichtung, das Schieben des Verschlußelements vor die Spitze der Kanüle und das Verrasten der Zunge am Basiselement.

Bei der Kanüle handelt es sich vorzugsweise um eine Port-Punktionsnadel für ein Portkathetersystem, insbesondere um eine Huber-Nadel. Vorzugsweise ist die Kanüle mit einem Anschluß für einen Schlauch versehen.

Vorzugsweise weist die Kanüle in der Nähe der Spitze einen Abschnitt mit einem Querschnitt auf, der gegenüber einem Kanülenschaft vergrößert ist. Dies ist beispielsweise bei einer Port-Nadel mit Huber-Schliff der Fall, die einen Kanülenschaft mit einem Durchmesser von 1,1 mm hat und an einer Biegung in der Nähe der Spitze einen maximalen Durchmesser von 1,4 mm hat. Vorzugsweise weist das Basiselement entfernt von der Unterseite des Basiselements eine Durchtrittsöffnung für den Kanülenschaft auf, die einen Querschnitt hat, durch den der Kanülenabschnitt mit vergrößertem Querschnitt nicht hindurchpaßt. Im genannten Beispiel ist die Durchtrittsöffnung zylindrisch mit einem Durchmesser von 1,2 mm. Die Durchtrittsöffnung und das Verschlußelement bzw. Betätigungselement sind so angeordnet, daß beim Zurückziehen der Spitze der Kanüle der Kanülenabschnitt mit vergrößertem Querschnitt an der Durchtrittsöffnung hängen bleibt, so daß die Spitze der Kanüle sich zwischen der Durchtrittsöffnung und dem Verschlußelement im inneren des Basiselements befindet. Somit wird ein sicherer Schutz vor Verletzungen erreicht.

Die Vorspanneinrichtung weist vorzugsweise eine zwischen dem Basiselement und dem Zugelement oder einem Deckel, an welchem das Zugelement angeordnet ist, angeordnete Druckfeder auf. Bei der Druckfeder handelt es sich beispielsweise um eine Schraubenfeder, vorzugsweise um eine spiralförmig verlaufende, beispielsweise kegelförmige Blattfeder, wobei jeweils die Hauptbelastungsrichtung der Druckfeder in Richtung der Kanüle verläuft. Vorzugsweise ist die Druckfeder ringförmig oder spiralförmig um die Mittelachse der Kanüle herum angeordnet. Vorzugsweise ist die Querausdehnung der Druckfeder bei vorgespannter Vorspanneinrichtung größer als ihre axiale Ausdehnung, insbesondere um ein mehrfaches größer als ihre axiale Ausdehnung. Dadurch ergibt sich ein flacher Aufbau der Vorrichtung.

Vorzugsweise ist vor einer Unterseite des Betätigungselements, also vor derjenigen Seite, an der die Kanüle in der gebrauchsfähigen Stellung hervorragt, ein Bodenelement, insbesondere eine Bodenplatte, des Basiselements angeordnet. Dieses bildet vorzugsweise eine Auflage zum Aufliegen der Vorrichtung auf die Haut des Patienten. Durch ein derartiges Bodenelement wird die Bewegung des Betätigungselements erleichtert, während die Vorrichtung an der Haut des Patienten anliegt, da das Betätigungselement nicht unmittelbar auf der Haut verschoben werden braucht.

Vorzugsweise ist das Betätigungselement in Längsrichtung der Kanüle beabstandet von einer Auflagefläche des Basiselements zum Auflegen der Vorrichtung auf die Haut eines Patienten.

Im folgenden werden Ausführungsbeispiele der Erfindung anhand der Zeichnung näher erläutert.

Es zeigen:
- Fig. 1: einen Schnitt durch eine erfindungsgemäße Vorrichtung mit einem Basiselement und einem Zugelement in Form eines Deckels, an welchem eine Kanüle gehalten ist;
- Fig. 2: eine Ansicht der Vorrichtung von unten mit abgenommener Bodenplatte, wobei die Linie I-I dem in Fig. 1 gezeigten Schnitt entspricht;
- Fig. 3: eine Explosionsdarstellung der Vorrichtung entsprechend Fig. 1;
- Fig. 4: eine Ansicht der Vorrichtung von unten mitsamt Bodenplatte;
- Fig. 5: eine Ansicht der Vorrichtung von oben;
- Fig. 6: einen Schnitt durch eine Vorrichtung gemäß einer zweiten Ausführungsform mit einem Basiselement und einem Zugelement in Form eines Deckels, an welchem eine Kanüle gehalten ist;
- Fig. 7: eine Ansicht des Basiselements der Vorrichtung aus Fig. 6 von unten, wobei die Linie VI - VI der dem in Fig. 6 gezeigten Schnitt entspricht; und
- Fig. 8: eine Seitenansicht des Zugelements und Deckels aus Fig. 6 mit der Kanüle.

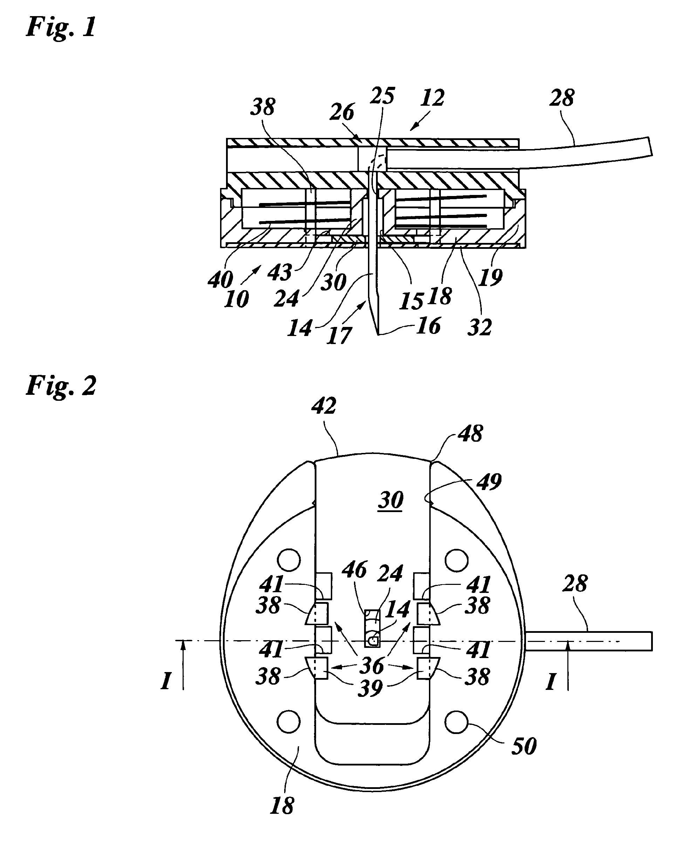

Die in Fig. 1 gezeigte Vorrichtung weist ein Gehäuse auf, das aus einem Basiselement 10 und einem Zugelement 12 besteht, welches zugleich einen Deckel des Gehäuses bildet. Das Basiselement 10 und das Zugelement 12 haben im wesentlichen einen zylindrischen Außenquerschnitt. Entlang ihrer gemeinsamen Mittelachse ist eine Kanüle 14 angeordnet, die durch eine Öffnung 15 in dem Basiselement 10 mit ihrer Spitze 16 hindurchragt und in der in Fig. 1 gezeigten gebrauchsfertigen Stellung der Vorrichtung so weit über die flache Unterseite des Basiselements 10 hinausragt, daß ein subkutan implantierter Port eines Portkathetersystems erreichbar ist. Die hinausragende Länge der Kanüle 14 ist beispielsweise so bemessen, daß bei in den Port eingeführter Kanüle 14 das Basiselement 10 mit seiner Unterseite auf der Haut des Patienten abgestützt werden kann. Die Kanüle 14 ist beispielsweise als Huber-Nadel ausgeführt und hat einen Kanülenschaft mit einem Durchmesser von 1,1 mm und oberhalb der Kanülenöffnung an einer Biegung einen Abschnitt 17 mit einem maximalen Durchmesser von 1,4 mm.

Das Basiselement 10 bildet einen flachen Topf 18 mit einer ringförmigen Seitenwand 19. In der Mitte des Topfes 18 ist oberhalb der Öffnung 15 eine innere, zylindrische Wand 24 ausgebildet, die an ihrem oberen Ende eine Durchtrittsöffnung für den Kanülenschaft in Form einer Verengung 25 mit einem Durchmesser von beispielsweise 1,2 mm aufweist.

Das Zugelement 12 bildet einen Deckel für den Topf 18 und trägt in seiner Mitte eine Halterung 26 für die Kanüle 14 und einen mit dieser flüssigkeitsdicht verbundenen Schlauch 28.

In einer Vertiefung an der Unterseite des Topfes 18 ist ein plattenförmiges Element 30 verschiebbar gelagert und wird an seiner Unterseite von einer Bodenplatte 32 des Basiselements 10 erhalten. In Figur 1 ist das Element 30 in Blickrichtung vor und zurück schiebbar.

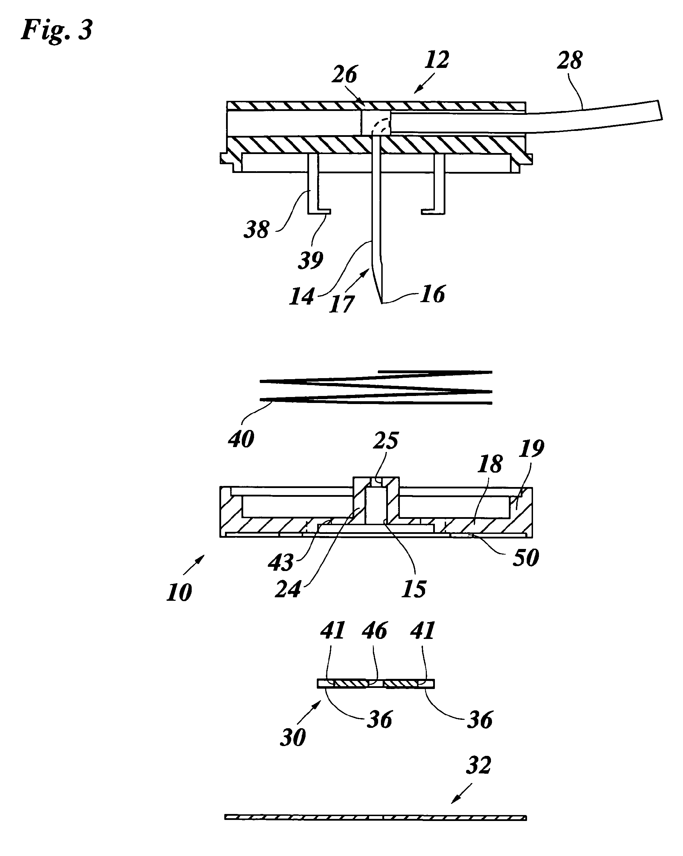

Figur 2 zeigt, daß das Betätigungselement Riegelelemente in Form von Bereichen 36 bildet. Gegenstücke 38 dazu sind an der Unterseite des Zugelements 12 in Form von beiderseits des Elements 30 nach unten vorstehenden Riegelelementen gebildet, die im wesentlichen L-förmig mit einem sich unter einem Bereich des Elements 30 erstreckenden Vorsprung 39 geformt sind. Dies ist insbesondere in Figur 2 und in Figur 3 zu sehen ist. Figur 3 zeigt zur besseren Veranschaulichung die Schnittansicht aus Figur 1 in einer Explosionsdarstellung.

In einer gebrauchsfertigen Stellung der Vorrichtung wird das Element 30 an den Bereichen 36 von den Gegenstücken 38 mit den Vorsprüngen 39 hintergriffen, so daß das Zugelement 12 über die Vorsprünge 39 an dem Element 30 und somit an dem Basiselement 10 verriegelt ist. In dieser Stellung ist das Zugelement 12 gegen das Basiselement 10 vorgespannt, indem eine Druckfeder 40 (Figur 1) vorgespannt zwischen dem Boden des Topfes 18 und dem Deckel des Zugelements 12 angeordnet ist. Auch Figur 3 zeigt die Druckfeder 40 im vorgespannten Zustand.

In Figur 2 ist zu sehen, daß das Element 30 neben den Bereichen 36 jeweils seitliche Aussparungen 41 am Rand aufweist. Das Element 30 weist an seinem vorderen Rand (in Figur 2 oben) eine Griffläche 42 auf. Die von den Riegelelementen 38, den mit ihr in Eingriff stehenden Bereichen 36 des Elements 30 und der Druckfeder 40 gebildete Vorspanneinrichtung wird durch Erfassen der Griffläche 42 und Hineindrücken des Elements 30 in Richtung auf die Mittelachse der Kanüle 14 gelöst, indem das Element 30 so verschoben wird, daß die Vorsprünge 39 in die Aussparungen 41 gelangen, so daß sie von den Bereichen 36 freigegeben werden. In dem Boden des Topfes 18 sind entsprechende Aussparungen 43 vorgesehen, die in Figur 1 gestrichelt angedeutet sind.

Aufgrund des Freigebens der Riegelelemente 38 wird das Zugelement 12 durch den Druck der Druckfeder 40 von dem Basiselement 10 abgehoben. Dabei treten die Vorsprünge 39 nach oben aus dem Boden des Topfes 18 aus. Die Griffläche 42 und das Element 30 bilden somit ein Betätigungselement zum Lösen der Vorspanneinrichtung.

Beim Abheben des Zugelements 12 von dem Basiselement 10 nimmt das Zugelement 12 die Kanüle 14 mit. Dadurch wird die Spitze 16 hinter die Öffnung 15 zurückgezogen. Das Element 30 weist ein hinreichend große Öffnung 46 auf, um das Verschieben des Elements 30 trotz hindurchtretender Kanüle 14 zu gestatten. An der Verengung 25 der Wand 24 bleibt die Kanüle mit ihrem Abschnitt 17 mit vergrößertem Querschnitt hängen, so daß die Spitze 16 im Inneren des Topfes 18, umgeben von der Wand 24, verborgen ist und so vor Berührung geschützt ist.

Wenn das Element 30 weitergeschoben wird, verschließt es schließlich die Öffnung 15, indem sich ein geschlossener Bereich des Elements 30 vor der in das Basiselement 10 zurückgezogenen Spitze 16 der Kanüle 14 befindet. An dem Element 30 sind seitlich kleine Rastnasen 48 gebildet, die bei vollständig eingeschobenem Element 30 an Rastaufnahmen 49 im Boden des Topfes 18 einrasten. Die Rastnasen 48 und die Rastaufnahmen 49 bilden zusammen eine Verriegelungseinrichtung für das Element 30, die ein Zurückschieben des Elements 30 verhindert. Dadurch wird die Öffnung 15 sicher verschlossen, so daß die Spitze 16 der Kanüle 14 nicht wieder austreten kann. Das Element 30 verbirgt somit die Spitze 16.

Bei der beschriebenen Vorrichtung wird also durch Erfassen der Griffläche 42 und gegebenenfalls der gegenüberliegenden Seite des Topfes 18 und Hineindrücken des Elements 30 erreicht, daß zunächst durch die Aussparungen 41 ein Freigeben der Riegelelemente 38 des Deckels des Zugelements 12 erfolgt und so die Vorspanneinrichtung gelöst wird und die Kanüle 14 in das Basiselement 10 zurückgezogen wird. Bei weiterem Hineindrücken des Elements 30 tritt dieses vor die zurückgezogene Spitze 16 der Kanüle 14 und verbirgt diese und wird in dieser Position durch die Verriegelungseinrichtung 48, 49 verriegelt.

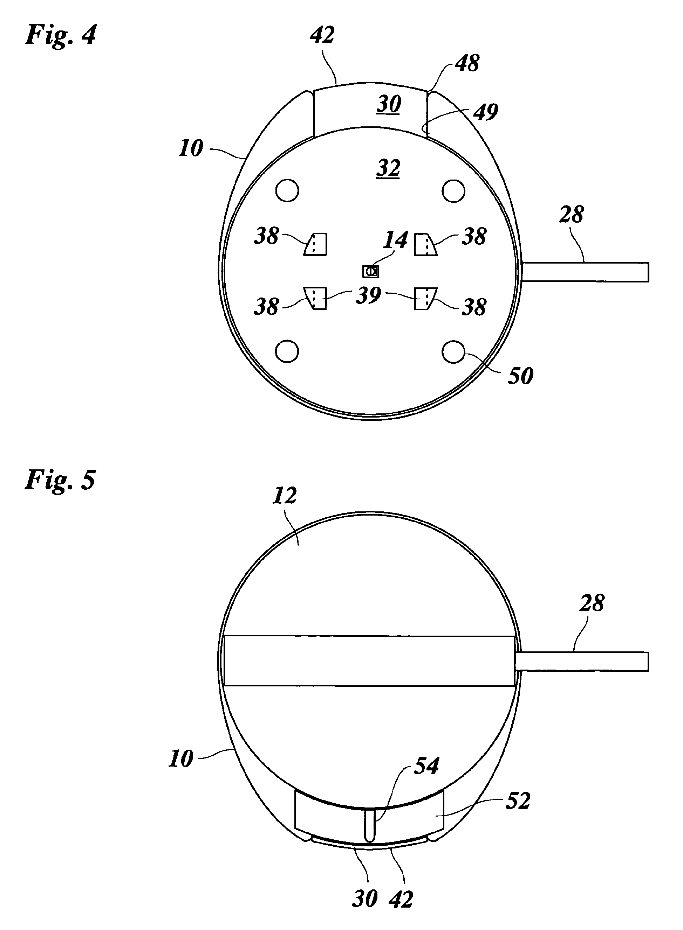

Figur 4 zeigt eine Ansicht entsprechend Figur 2, jedoch mit aufgesetzter Bodenplatte 32, die über vier Vorsprünge 50 an der Unterseite des Topfes 18 und entsprechende Aussparungen in der Bodenplatte 32 ausgerichtet ist. Die Bodenplatte 32 weist ebenfalls Aussparungen für die Riegelelemente 38 mit den Vorsprüngen 39 auf, so daß ein besonders flacher Aufbau erreicht wird.

Figur 5 zeigt eine Ansicht der Vorrichtung von oben, wobei zwischen einem nach oben vorspringenden Rand des Elements 30, an dem die Griffläche 42 ausgebildet ist, und der Außenwand des Topfes 18 ein Sicherungselement 52 eingesetzt ist. Das Sicherungselement 52 kann beispielsweise zwischen einem nach oben vorspringenden Rand des Elements 30 und der Wand des Topfes 18 eingeklemmt sein. Es dient dazu, ein versehentliches Hineinschieben des Elements 30 zu verhindern. Das Sicherungselement 52 kann durch Erfassen eines Griffs 54 entfernt werden, wenn die Vorrichtung betätigt werden soll.

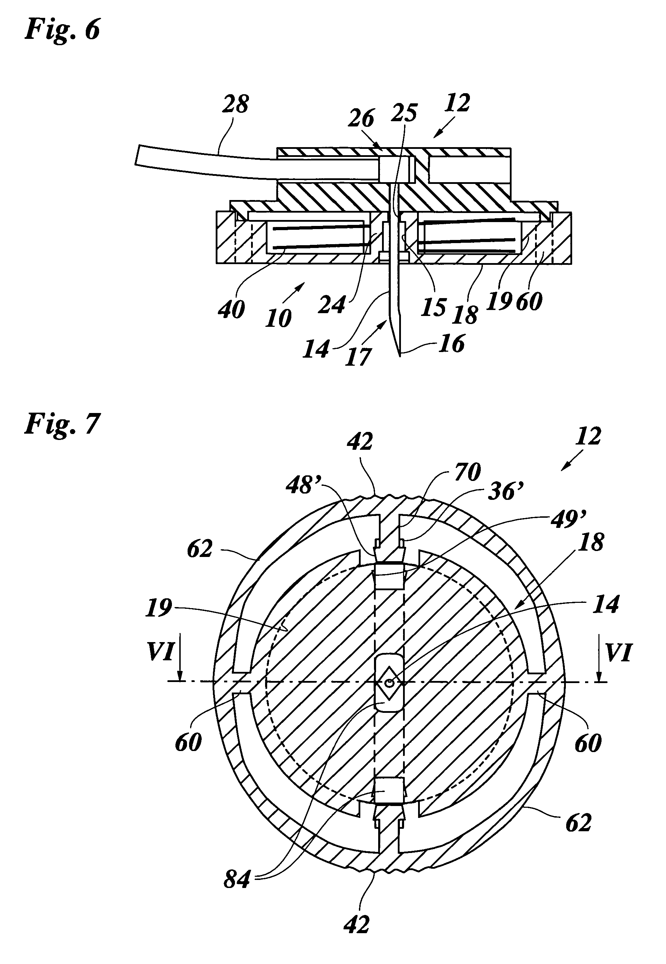

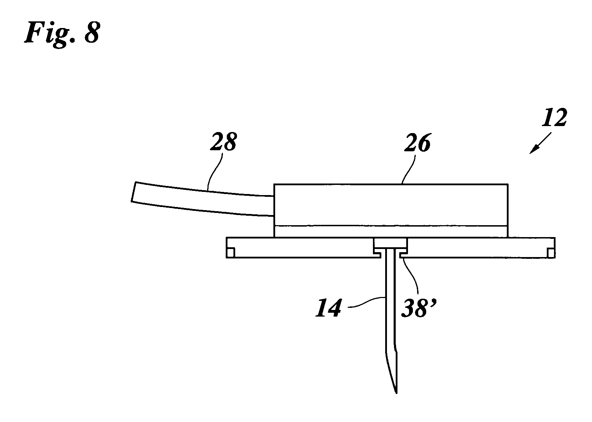

Figuren 6 bis 8 zeigen eine zweite Ausführungsform der Vorrichtung, die sich hinsichtliche der Anzahl und Art der Betätigungselemente sowie des Aufbaus der Verriegelungsvorrichtung für die Vorspanneinrichtung von der in den Figuren 1 bis 5 gezeigten Ausführungsform unterscheidet. Einander entsprechende Elemente sind mit den gleichen Bezugszeichen gekennzeichnet.

Die Seitenwand 19 des Topfes 18 ist in den Figuren 6 und 7 gestrichelt angedeutet, und der Topf 18 ist mit Abstand von einem ringförmigen Element umgeben, welches an zwei gegenüberliegenden Seiten über Stege 60 mit dem Topf 18 verbunden ist und auf diese Weise in zwei halbringförmige Bügel 62 unterteil ist.

Figur 7 zeigt, daß an jedem Bügel 62 in der Mitte zwischen den Stegen 60 eine nach innen zum Topf 18 gerichtete Zunge 70 angeformt ist. Die Zunge 70 bildet an ihrem vorderen Ende eine harpunenförmige Verbreiterung 48', die mit einer geeignet geformten Rastaufnahme 49' zusammenwirkt. Beim Hineindrücken der Zunge 70 in die Rastaufnahme 49' rastet die Verbreiterung 48' in der Rastaufnahme 49' ein, so daß die Zunge 70 festgehalten wird.

Hinter der Verbreiterung 48' sind an der Zunge 70 Riegelelemente 36' in Form von seitlichen Vorsprüngen ausgebildet. Diese Riegelelemente 36' sind an der Oberseite der Zunge 70 angeordnet. Am Rand des Deckels des Zugelements 12 sind entsprechende Riegelelemente angeordnet, die Gegenstücke 38' zu den Riegelelementen 36' bilden und in der gebrauchsfertigen Stellung so an den Riegelelementen 36' gehalten sind, daß das Zugelement 12 an den Zungen 70 und somit an dem Basiselement 10 verriegelt ist. In dieser Stellung ist das Zugelement 12 wiederum gegen das Basiselement 10 vorgespannt, indem die Druckfeder 40 (Fig. 6) vorgespannt zwischen dem Boden des Topfes 18 und dem Deckel des Zugelements 12 angeordnet ist.

Die Bügel 62 weisen an der jeweils der Zunge 70 gegenüberliegenden Außenseite eine Griffläche 42 auf. Die von den Riegelelementen 36', ihren Gegenstücken 38' und der Druckfeder 40 gebildete Vorspanneinrichtung wird durch Erfassen der sich gegenüberliegenden Grifflächen 42 und Hineindrücken der Zungen 70 in Richtung auf die Mittelachse der Kanüle 14 gelöst, indem die Riegelelemente 36' nach radial innen bewegt werden und dabei die Gegenstücke 38' freigeben, so daß das Zugelement 12 durch den Druck der Druckfeder 40 von dem Basiselement 10 abgehoben wird. Die Grifflächen 42 und die Zungen 70 bilden somit Betätigungselemente zum Lösen der Vorspanneinrichtung. In gleicher Weise wie bei der ersten Ausführungsform wird beim Abheben des Zugelement 12 von dem Basiselement 10 die Kanüle 14 mitgenommen, und die Spitze wird im Inneren des Topfes 18, umgeben von der Wand 24, verborgen.

Im Boden des Topfes 18 sind einander gegenüberliegend zwei Verschlußelemente in Form von Verschlußblechen 84 verschiebbar angeordnet. Die Verschlußbleche 84 liegen auf der durch die Kanüle 14 gehenden gedachten Verbindungslinie der beiden Zungen 70 und sind parallel zu dieser verschiebbar. Die Verschlußbleche 84 erstrecken sich nach außen jeweils bis kurz vor die Zunge 70, so daß sie beim Hineindrücken der Zunge 70 in die Rastaufnahme 49' in Richtung der Mittelachse der Kanüle 14 vorgeschoben werden. Wenn die Zunge 70 mit der Verbreiterung 48' an der Rastaufnahme 49' verriegelt ist, befindet sich das jeweilige vordere Ende des Verschlußbleche 84 vor der in das Basiselement 10 zurückgezogenen Spitze 16 der Kanüle 14. Die Zunge 70 bildet somit einen Stößel zum Bewegen des Verschlußblechs 84 nach Innen vor die Spitze 16 der zurückgezogenen Kanüle 14. Die Verbreiterung 48' und die Rastaufnahme 49' bilden zusammen eine Verriegelungseinrichtung für das Verschlußblech 84, die ein Zurückschieben des Verschlußblechs 84 verhindert. Dadurch wird die Öffnung 15 sicher verschlossen, so daß die Spitze 16 der Kanüle 14 nicht wieder austreten kann. Die Verschlußbleche 84 verbergen somit die Spitze 16.

Bei der zweiten Ausführungsform der Vorrichtung wird also durch Erfassen der Grifflächen 42 und Zusammendrücken der Bügel 62 erreicht, daß durch das Hineindrücken der Zungen 70 zunächst die Riegelelemente 36' den Deckel des Zugelements 12 freigeben und so die Vorspanneinrichtung lösen und die Kanüle 14 in das Basiselement 10 zurückgezogen wird, woraufhin bei weiterem Hineindrücken der Zungen 70 die Verschlußbleche 84 vor die Spitze 16 der Kanüle 14 treten und diese verbergen und in dieser Position durch die Verriegelungseinrichtung 48', 49' verriegelt werden.

## Patentansprüche

1. Vorrichtung zum Applizieren einer Kanüle (14), aufweisend:
- ein Basiselement (10),
- eine Kanüle (14), die mit ihrer Spitze (16) in einer gebrauchsfertigen Stellung der Vorrichtung über das Basiselement (10) hinausragt,
- ein gegenüber dem Basiselement (10) bewegliches Zugelement (12) und eine lösbare Vorspanneinrichtung (36, 38, 40) zum Vorspannen des Zugelements (12) gegenüber dem Basiselement (10) in der gebrauchsfertigen Stellung der Vorrichtung,
- wenigstens ein Betätigungselement (30, 42) zum Lösen der Vorspanneinrichtung (36, 38, 40), wobei das Betätigungselement (30, 42) durch Drücken des Betätigungselements (30, 42) in eine Richtung quer zur Kanüle (14) betätigbar ist, wobei das Betätigungselement (30, 42) seitlich am Basiselement (10) angeordnet ist zum Betätigen durch Drücken des Betätigungselements (30, 42) in Richtung zur Mittelachse der Kanüle (14), und
- wenigstens ein Verschlußelement (30; 84) zum Verbergen der Spitze (16) der Kanüle (14), wenn diese in das Basiselement (10) zurückgezogen wurde, wobei das Verschlußelement (30; 84) bewegbar an dem Basiselement (10) gehalten ist,
wobei das Zugelement (12) derart mit der Kanüle (14) zusammenwirkt, daß bei einem Lösen der Vorspanneinrichtung (36, 38, 40) das Zugelement (12) die Spitze (16) der Kanüle (14) in das Basiselement (10) zurückzieht,
**dadurch gekennzeichnet,**
**dass** durch Drücken des Betätigungselements (30, 42) in der Richtung zur Mittelachse der Kanüle (14) das Verschlußelement (30; 84) vor die in das Basiselement (10) zurückgezogene Spitze (16) der Kanüle (14) bewegbar ist.

2. Vorrichtung nach Anspruch 1, **dadurch gekennzeichnet, daß** die Vorrichtung für Einhandbedienung zum Festhalten des Basiselements (14) und Lösen der Vorspanneinrichtung (36, 38, 40), während das Basiselement (14) auf der Haut eines Patienten anliegt, ausgelegt ist.

3. Vorrichtung nach Anspruch 1 oder 2, wobei an dem Betätigungselement (30, 42) ein Riegelelement (36) angeordnet ist und an dem Zugelement (12) ein Gegenstück (38) zu dem Riegelelement (36) angeordnet ist, welches in der gebrauchsfertigen Stellung an dem Riegelelement (36) gehalten ist, und das Lösen der Vorspanneinrichtung (36, 38, 40) erfolgt, indem das Riegelelement (36) das Gegenstück (38) freigibt.

4. Vorrichtung nach einem der vorstehenden Ansprüche, wobei das Betätigungselement (30, 42) plattenförmig ist und an dem Basiselement (10) gehalten ist und in der gebrauchsfertigen Stellung das Zugelement (12) an dem Betätigungselement (30, 42) verriegelt ist, wobei die Verriegelung durch Bewegen des Betätigungselements (30, 42) in Richtung zur Mittelachse der Kanüle (14) lösbar ist.

5. Vorrichtung nach einem der vorstehenden Ansprüche, bei der das Betätigungselement (30, 42) an dem Basiselement (10) in einer Richtung quer zur Kanüle (14) bewegbar gehalten ist und in der gebrauchsfertigen Stellung wenigstens ein Riegelelement (38) des Zugelements (12) ein Riegelelement (36) des Betätigungselements (30, 42) in Form eines Bereichs des Betätigungselements (30, 42) hintergreift, welcher Bereich geformt ist, um durch Bewegen des Betätigungselements (30, 42) in Richtung zur Mittelachse der Kanüle (14) außer Eingriff mit dem Riegelelement (38) des Zugelements (12) gebracht zu werden.

6. Vorrichtung nach einem der vorstehenden Ansprüche, mit einer Verriegelungseinrichtung (48, 49) zum Verriegeln des Verschlußelements (30, 42) in der die Spitze (16) verbergenden Stellung.

7. Vorrichtung nach einem der vorstehenden Ansprüche, wobei die Kanüle (14) in der Nähe der Spitze (16) einen Abschnitt (17) mit einem Querschnitt aufweist, der gegenüber dem Querschnitt eines Schafts der Kanüle (14) vergrößert ist, und wobei das Basiselement (10) entfernt von einer Unterseite des Basiselements (10), an welcher Unterseite in der gebrauchsfertigen Stellung die Kanüle (14) mit ihrer Spitze (16) hinausragt, eine Durchtrittsöffnung (25) für den Schaft der Kanüle (14) aufweist, die einen Querschnitt hat, durch den der Kanülenabschnitt (17) mit vergrößertem Querschnitt nicht hindurchpaßt.

8. Vorrichtung nach einem der vorstehenden Ansprüche, wobei die Kanüle (14) eine Port-Punktionsnadel für ein Portkathetersystem ist.

9. Vorrichtung nach einem der vorstehenden Ansprüche, wobei das Verschlußelement (30; 84) an einem Federelement angeordnet ist und in einer gebrauchsfertigen Stellung vorgespannt ist und durch Betätigen des Betätigungselements (30, 42) auslösbar ist, um durch die Federkraft des Federelements eine Stellung vor der in das Basiselement (10) zurückgezogenen Spitze (16) der Kanüle (14) einzunehmen.

10. Vorrichtung nach einem der Ansprüche 1 bis 8, wobei das Betätigungselement (30) das Verschlußelement bildet.

11. Vorrichtung nach einem der Ansprüche 1 bis 8, wobei das Betätigungselement (42) einen Stößel aufweist oder mit einem Stößel verbunden ist oder einen Stößel (70) steuert, welcher Stößel (70) das Verschlußelement (84) verschiebt.

## Claims

1. A device for administering a cannula (14), comprising:
- a basic element (10),
- a cannula (14), the tip (16) of which protrudes beyond the basic element (10) in a ready to use position of the device,
- a traction element (12), which is movable with respect to the basic element (10), and a releasable pretensioning device (36, 38, 40) for pretensioning the traction element (12) with respect to the basic element (10) in the ready to use position of the device,
- at least one actuating element (30, 42) for releasing the pretensioning device (36, 38, 40), wherein the actuating element (30, 42) can be actuated by pressing the actuating element (30, 42) in a direction at right angles to the cannula (14), wherein the actuating element (30, 42) is arranged at the side of the basic element (10) for being actuated by pressing the actuating element (30, 42) in the direction towards the central axis of the cannula (14), and
- at least one closing element (30; 84) for hiding the tip (16) of the cannula (14), when this tip has been retracted into the basic element (10), wherein the closing element (30; 84) is moveably held at the basic element (10),
wherein the traction element (12) interacts with the cannula (14) such that, when the pretensioning device (36, 38, 40) is released, the traction element (12) retracts the tip (16) of the cannula (14) into the basic element (10),
**characterized in**
**that** the closing element (30; 84) can be moved in front of the tip (16) of the cannula (14), retracted into the basic element (10), by pressing the actuating element (30, 42) in the direction towards the central axis of the cannula (14).

2. The device according to claim 1, **characterized in that** the device is designed for a one-handed operation for holding the basic element (14) and releasing the pretensioning device (36, 38, 40), while the basic element (14) is resting on the skin of a patient.

3. The device according to claim 1 or 2, wherein a locking element (36) is disposed at the actuating element (30, 42) and a counter piece (38) of the locking element (36), which is held at the locking element (36) in the ready to use position, is disposed at the traction element (12), and the pretensioning device (36, 38, 40) is released in that the locking element (36) releases the counter piece (38).

4. The device according to any one of the preceding claims, wherein the actuating element (30, 42) is plate-like and held at the basic element (10) and the traction element (12) is locked at the actuating element (30, 42) in the ready to use position, wherein the locking can be released by moving the actuating element (30, 42) in the direction towards the central axis of the cannula (14).

5. The device according to any one of the preceding claims, wherein the actuating element (30, 42) is held at the basic element (10) movably in a direction at right angles to the cannula (14) and, in the ready to use position, at least one locking element (38) of the traction element (12) engages from the rear a locking element (36) of the actuating element (30, 42) in the form of a region of the actuating element (30, 42), this region being shaped so that it is disengaged from the locking element (38) of the traction element (12) by moving the actuating element (30, 42) in the direction towards the central axis of the cannula (14).

6. The device according to any one of the preceding claims, with a locking device (48, 49) for locking the closing element (30, 42) in the position hiding the tip (16).

7. The device according to any one of the preceding claims, wherein, in the vicinity of the tip (16), the cannula (14) has a section (17) with a cross-section, which is larger than the cross-section of the shaft of the cannula (14), and wherein the basic element (10), at a place remote from an underside of the basic element (10), at which underside the cannula (14), in the ready to use position, protrudes with its tip (16), has a passage opening (25) for the shaft of the cannula (14), and the passage opening has a cross-section, through which the section (17) of the cannula (14) with the larger cross-section cannot pass.

8. The device according to any one of the preceding claims, wherein the cannula (14) is a port puncture needle for a port catheter system.

9. The device according to any one of the preceding claims, wherein the closing element (30; 84) is disposed at a spring element and is pretensioned in a ready to use position and can be actuated by actuating the actuating element (30, 42), in order to assume, by the spring force of the spring element, a position in front of the tip (16) of the cannula (14), retracted into the basic element.

10. The device according to any one of claims 1 to 8, wherein the actuating element (30) forms the closing element.

11. The device according to any one of claims 1 to 8, wherein the actuating element (42) has a finger or is connected with a finger or controls a finger (70), which finger (70) shifts the closing element (84).

## Revendications

1. Dispositif de mise en place d'une canule (14), comportant :
- un élément de base (10),
- une canule (14) qui fait saillie avec sa pointe (16) au-delà de l'élément de base (10) dans une position prête à l'emploi du dispositif,
- un élément de traction (12) mobile par rapport à l'élément de base (10) et un dispositif de précontrainte libérable (36, 38, 40) pour précontraindre l'élément de traction (12) par rapport à l'élément de base (10) dans la position prête à l'emploi du dispositif,
- au moins un élément d'actionnement (30, 42) pour libérer le dispositif de précontrainte (36, 38, 40), dans lequel l'élément d'actionnement (30, 42) peut être actionné en pressant l'élément d'actionnement (30, 42) dans une direction transversale à la canule (14), dans lequel l'élément d'actionnement (30, 42) est agencé latéralement sur l'élément de base (10) de manière à être actionné en pressant l'élément d'actionnement (30, 42) en direction de l'axe central de la canule (14), et
- au moins un élément de fermeture (30 ; 84) pour cacher la pointe (16) de la canule (14), lorsque celle-ci a été rétractée dans l'élément de base (10), dans lequel l'élément de fermeture (30 ; 84) est maintenu mobile sur l'élément de base (10),
dans lequel l'élément de traction (12) coopère avec la canule (14) de sorte que lors d'une libération du dispositif de précontrainte (36, 38, 40), l'élément de traction (12) rétracte la pointe (16) de la canule (14) dans l'élément de base (10),
**caractérisé en ce que**
en pressant l'élément d'actionnement (30, 42) dans la direction de l'axe central de la canule (14), l'élément de fermeture (30 ; 84) peut être déplacé avant la pointe (16) de la canule (14) rétractée dans l'élément de base (10).

2. Dispositif selon la revendication 1, **caractérisé en ce que** le dispositif est conçu pour une manoeuvre à une main afin de tenir l'élément de base (10) et de libérer le dispositif de précontrainte (36, 38, 40) pendant que l'élément de base (10) repose sur la peau d'un patient.

3. Dispositif selon la revendication 1 ou 2, dans lequel un élément de blocage (36) est agencé sur l'élément d'actionnement (30, 42) et une pièce complémentaire (38) à l'élément de blocage (36) est agencée sur l'élément de traction (12), laquelle pièce complémentaire est maintenue sur l'élément de blocage (36) dans la position prête à l'emploi, et le dispositif de précontrainte (36, 38, 40) est libéré lorsque l'élément de blocage (36) libère la pièce complémentaire (38).

4. Dispositif selon l'une des revendications précédentes, dans lequel l'élément d'actionnement (30, 42) est en forme de plaque et est maintenu sur l'élément de base (10) et dans la position prête à l'emploi, l'élément de traction (12) est bloqué sur l'élément d'actionnement (30, 42), dans lequel le déblocage peut être effectué en déplaçant l'élément d'actionnement (30, 42) en direction de l'axe central de la canule (14).

5. Dispositif selon l'une des revendications précédentes, dans lequel l'élément d'actionnement (30, 42) est maintenu de manière mobile sur l'élément de base (10) dans une direction transversale à la canule (14) et dans la position prête à l'emploi, au moins un élément de blocage (38) de l'élément de traction (12) s'engage par l'arrière avec un élément de blocage (36) de l'élément d'actionnement (30, 42) ayant la forme d'une région de l'élément d'actionnement (30, 42), laquelle région est formée afin d'être désengagée de l'élément de blocage (38) de l'élément de traction (12) en déplaçant l'élément d'actionnement (30, 42) en direction de l'axe central de la canule (14).

6. Dispositif selon l'une des revendications précédentes, comportant un dispositif de blocage (48, 49) pour bloquer l'élément de fermeture (30, 42) dans la position cachant la pointe (16).

7. Dispositif selon l'une des revendications précédentes, dans lequel la canule (14), à proximité de la pointe (16), comporte une portion (17) ayant une section transversale qui est agrandie par rapport à la section transversale d'une tige de la canule (14), et dans lequel l'élément de base (10), éloigné d'une face inférieure de l'élément de base (10), face inférieure sur laquelle la canule fait saillie, dans la position prête à l'emploi, avec sa pointe, comporte une ouverture de passage (25) pour la tige de la canule (14), laquelle ouverture de passage a une section transversale à travers laquelle la portion de canule (17) ayant une section transversale agrandie ne passe pas.

8. Dispositif selon l'une des revendications précédentes, dans lequel la canule (14) est une aiguille de ponction à orifices pour un système de cathéter à orifices.

9. Dispositif selon l'une des revendications précédentes, dans lequel l'élément de fermeture (30 ; 84) est agencé sur un élément à ressort et est précontraint dans une position prête à l'emploi, et peut être libéré en actionnant l'élément d'actionnement (30, 42) afin de prendre une position devant la pointe (16) de la canule (14) rétractée dans l'élément de base (10) par la force élastique de l'élément à ressort.

10. Dispositif selon l'une des revendications 1 à 8, dans lequel l'élément d'actionnement (30) forme l'élément de fermeture.

11. Dispositif selon l'une des revendications 1 à 8, dans lequel l'élément d'actionnement (42) comporte un poussoir ou est relié à un poussoir ou commande un poussoir (70), lequel poussoir (70) déplace l'élément de fermeture (84).
